(19) Europäisches Patentamt
European Patent Office
Office européen des brevets

(11) **EP 4 628 140 A1**

(12) **EUROPEAN PATENT APPLICATION**
published in accordance with Art. 153(4) EPC

(43) Date of publication:
**08.10.2025 Bulletin 2025/41**

(21) Application number: **23896460.5**

(22) Date of filing: **07.11.2023**

(51) International Patent Classification (IPC):
*A61M 25/01* (2006.01)    *A61B 34/30* (2016.01)
*A61B 34/10* (2016.01)

(86) International application number:
**PCT/CN2023/130041**

(87) International publication number:
**WO 2024/114308 (06.06.2024 Gazette 2024/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  28.11.2022  CN 202211497241
28.11.2022  CN 202211497242

(71) Applicant: Shenzhen Edge Medical Co., Ltd.
Shenzhen, Guangdong 518000 (CN)

(72) Inventors:
• LUO, Xinggui
Shenzhen, Guangdong 518000 (CN)
• XIAO, Fan
Shenzhen, Guangdong 518000 (CN)
• GAO, Yuanqian
Shenzhen, Guangdong 518000 (CN)

(74) Representative: Metida
Gyneju str. 16
01109 Vilnius (LT)

(54) **CATHETER BENDING STEERING CONTROL METHOD, CATHETER SYSTEM, AND STORAGE MEDIUM**

(57)    Provided are a catheter bending steering control method, a catheter system, and a readable storage medium. The method comprises: acquiring a position change amount of the tail end of a catheter according to a catheter steering instruction from a master controller (S11), the position change amount comprising a direction angle and a bending angle; determining, according to the position change amount, driving wheels and driven wheels in a plurality of driving wheels, and calculating an angular position change amount of the driving wheel (S12); calculating an angular position change amount of the driven wheel according to the angular position change amount of the driving wheel and a current position proportion allocation coefficient of the corresponding driven wheel (S13); and controlling a driving motor to drive the corresponding driving wheel to rotate according to the angular position change amount (S14). By means of the described manner, bending steering control of the catheter can be achieved, the calculation process of the angular position change amount is simplified, and the response time of the catheter steering instruction is shortened.

Acquiring a position change amount of an end of a catheter according to a catheter steering instruction from a main controller — S11

Determining driving wheels and driven wheels in drive wheels according to the position change amount, and calculating an angular position change amount of the driving wheel — S12

Calculating an angular position change amount of the driven wheel according to the angular position change amount of the driving wheel and a current position proportion allocation coefficient of the corresponding driven wheel — S13

Controlling a drive motor to drive the corresponding drive wheel to rotate according to the angular position change amount — S14

FIG.4

**Description**

This application claims priority to the Chinese patent application with application number "202211497241.2" filed on November 28, 2022, and invention name "Catheter bending steering control method, catheter system and storage medium". This application claims priority to the Chinese patent application with application number "2022114972427" filed on November 28, 2022, and invention name "Catheter bending steering control method, catheter system and storage medium". The entire contents of these texts are incorporated into this application by reference.

TECHNICAL FIELD

The present disclosure relates to medical apparatus and instruments technologies field, in particular to a catheter bending steering control method, a catheter system, and computer-readable storage medium.

BACKGROUND

Minimally invasive medical techniques are intended to reduce the amount of tissue damaged during medical procedures to reduce patient recovery time, discomfort, and harmful side effects. In minimally invasive medical techniques, it is often necessary to insert a catheter through a natural orifice in the patient's anatomy or through a surgical incision to the target tissue location. In order for the catheter to reach the target tissue location, precise control of the catheter's steering is required.

In the related art, a catheter kinematic model is generally established, that is, the mapping relationship between the angular position of each driving wheel controlling the catheter and the catheter end posture is found, and then the target posture of the catheter is substituted into the kinematic model to solve the angular position of the driving wheel to achieve precise control of the catheter. Since the catheter is a flexible continuum structure, in actual applications, the material properties, assembly process, temperature changes, external force disturbances and working space of the catheter have a great interference with the kinematic description, resulting in a large deviation between the established kinematic model and the actual motion state, making it difficult to meet the requirements of real-time and precise control of the catheter steering.

SUMMARY OF THE INVENTION

The present application proposes a catheter bending steering control method, a catheter system and a computer-readable storage medium, which can solve the problem in the related art that it is difficult to meet the demand for real-time and precise control of catheter steering.

To achieve the above objectives, the present application provides a catheter system. The catheter system includes a mechanical arm, a catheter instrument engaged with a power unit of the mechanical arm, a main controller and a processor communicatively connected to the mechanical arm, the catheter instrument comprising an instrument box configured to be engaged with the power unit and a catheter connected to the instrument box, the instrument box comprising drive wheels configured to be driven by the power unit and drive wires having one end wound around the drive wheels and the other end extending along the catheter and fixed to an end of the catheter, the power unit comprising a plurality of drive motors, the drive motors, the drive wheels and the drive wires correspond one to one. The processor is configured to perform the following steps: acquiring a position change amount of the end of the catheter according to a catheter steering instruction from the main controller, the position change amount comprising a direction angle and a bending angle; determining the driving wheel and the driven wheel in the drive wheels according to the position change amount, and calculating an angular position change amount of the driving wheel; calculating an angular position change amount of the driven wheel according to the angular position change amount of the driving wheel and a current position proportion allocation coefficient of the corresponding driven wheel, the current position proportion allocation coefficient configured to indicate a proportion of a reeling length of the driving wheel to a corresponding release length of the driven wheel in a current state; controlling the drive motor to drive the corresponding drive wheel to rotate according to the angular position change amount.

The catheter bending steering control method, the catheter system and the computer-readable storage medium provided by the present application, acquiring a position change amount of the end of the catheter according to a catheter steering instruction from a main controller, the position change amount comprising a direction angle and a bending angle; determining driving wheel and driven wheel in drive wheels according to the position change amount, and calculating an angular position change amount of the driving wheel, the drive wheels, drive motors, and drive wires correspond one to one, the drive wheels wound with corresponding drive wires and driven by the corresponding drive motors, the other end of the drive wire extends along the catheter and is fixed to the end of the catheter; calculating an angular position change amount of the driven wheel according to the angular position change amount of the driving wheel and a current position proportion allocation coefficient of the corresponding driven wheel, the current position proportion allocation coefficient configured to indicate a proportion of a reeling length of the driving wheel to a corresponding release length of the driven wheel in a current state; controlling the drive motor to drive the corresponding drive wheel to rotate according to the angular position change amount. The control is performed based on the position change amount under the action of the catheter steering instruction rather than the target position, and the drive wheels are divided into the driving wheels and the driven wheels, only the angular position change amount of the driving wheel is directly calculated according to the position change amount, and the angular position change amount of the driven wheel is calculated according to the angular position change amount of the driving wheel, thereby realizing the decoupling of the calculation of the angular position change amount of the driven wheel and the catheter steering instruction, thereby simplifying the calculation process of the angular position change amount of each drive wheel while realizing the bending steering control of the catheter, and shortening the response time of the catheter steering instruction.

BRIEF DESCRIPTION OF THE DRAWINGS

Various other advantages and benefits will become apparent to those of ordinary skill in the art by reading the detailed description of the preferred embodiments below. The accompanying drawings are only for the purpose of illustrating the preferred embodiments and are not to be considered as limiting the present application. Also, the same reference symbols are used throughout the accompanying drawings to represent the same components. In the accompanying drawings:

FIG. 1 is a schematic diagram of a catheter system provided in an embodiment of the present application;
FIG. 2 is a schematic diagram of a catheter device and a power unit provided in an embodiment of the present application;
FIG. 3 is a schematic diagram of the catheter device provided in an embodiment of the present application;
FIG. 4 is a flow chart of a catheter bending steering control method provided in an embodiment of the present application;
FIG. 5 is a schematic diagram of determining driving wheels and driven wheels according to a direction angle when n=3 and drive wires are evenly distributed in an embodiment of the present application;
FIG. 6 is a schematic diagram of determining the driving wheel and the driven wheel according to the direction angle when n=4 and the drive wires are evenly distributed in one embodiment of the present application;
FIG. 7 is a flow chart of calculating the angular position change amount of the driving wheel in one specific embodiment of the present application;
FIG. 8 is a schematic diagram of a kinematic model established for a rotating section in one embodiment of the present application;
FIG. 9 is a flow chart of calculating the angular position change amount of the driving wheel in another specific embodiment of the present application;
FIG. 10 is a flow chart of calculating the angular position change amount of the driving wheel in another specific embodiment of the present application;
FIG. 11 is a flow chart of the catheter bending steering control method provided in another embodiment of the present application;
FIG. 12 is a schematic diagram of a joint space division of the drive wheel and a corresponding control strategy in another specific embodiment of the present application;
FIG. 13 is a structural schematic diagram of a control system of the catheter system provided in one embodiment of the present application;
FIG. 14 is a schematic diagram of a computer-readable storage medium provided in one embodiment of the present application.

DETAILED DESCRIPTION

The exemplary embodiments of the present application will be described in more detail below with reference to the accompanying drawings. Although the exemplary embodiments of the present application are shown in the accompanying drawings, it should be understood that the present application can be implemented in various forms and should not be limited by the embodiments described herein. On the contrary, these embodiments are provided to enable a more thorough understanding of the present application and to fully convey the scope of the present application to those skilled in the art.
It should be noted that, unless otherwise specified, the technical terms or scientific terms used in the present application should be the common meanings understood by those skilled in the art to which the present application belongs.

A catheter bending steering control method, a catheter system and a control system thereof, and a computer-readable storage medium provided according to the embodiments of the present application are described below in conjunction with the accompanying drawings.

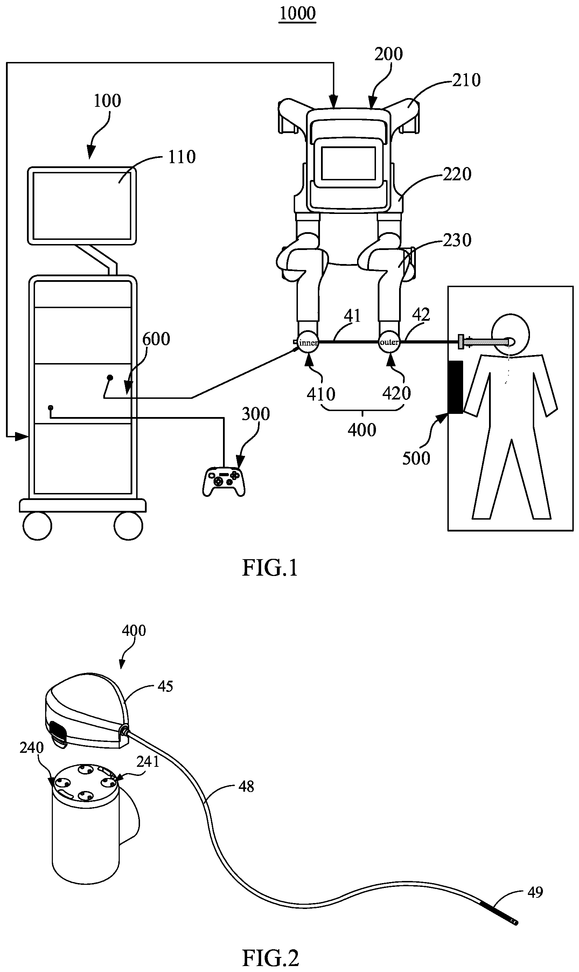

FIG. 1 illustrates a catheter system 1000 provided in an embodiment of the present application. The catheter system 1000 includes an imaging vehicle 100, a trolley 200 and a main controller 300 respectively connected to the imaging vehicle 100, a catheter instrument 400 that can be coupled (i.e., detachably connected) to the trolley 200, a sensor system 500 connected to the trolley 200, and a control system 600 for realizing control between the catheter instrument 400, the main controller 300, the sensor system 500 and the imaging vehicle 100. Among them, the main controller 300 can be connected to the trolley 200 by wire or wirelessly. When an operator performs various procedures on a patient next to the trolley 200, the operator can trigger control commands by operating the main controller 300, and control the catheter instrument 400 to forward, retract, bend, turn, etc. through the drive of the trolley 200.

The trolley 200 can usually be moved to a side of an operating bed to engage the catheter instrument 400, and control the catheter instrument 400 to move up and down in the vertical direction, or translate in the horizontal direction, or move in non-vertical and non-horizontal directions under the control command, so as to provide a better preoperative preparation angle for the operation of the catheter instrument 400. The control command can be a command triggered by the operator by operating the main controller 300, or a command triggered by the operator directly clicking or pressing a button set on the trolley 200. Of course, in other embodiments, the control command can also be a command triggered by voice control or a force feedback mechanism.

As shown in FIG. 1, further, the trolley 200 may include a base 210, a sliding seat body 220 that can be lifted and moved along the base 210, and two mechanical arms 230 fixedly connected to the sliding seat body 220. The mechanical arm 230 may include a plurality of arm segments connected at joints, and the plurality of arm segments provide the mechanical arm 230 with a plurality of degrees of freedom, for example, seven degrees of freedom corresponding to the seven arm segments. An end of the mechanical arm 230 is equipped with a power unit (not shown in the figure), and the power unit of the mechanical arm 230 is used to engage the catheter instrument 400, and under the driving action of the power unit, the end of the catheter instrument 400 is controlled to bend and turn accordingly. Among them, the two mechanical arms 230 can be completely identical in structure or partially identical in structure, one mechanical arm 230 is used to engage an inner catheter instrument 410, and the other mechanical arm 230 is used to engage an outer catheter instrument 420. During installation, the outer catheter instrument 420 can be installed first, and when the outer catheter instrument 420 is installed, the catheter of the inner catheter instrument 410 is inserted into the catheter of the outer catheter instrument 420.

The sensor system 500 has one or more subsystems for receiving information about the catheter device 400. The subsystems may include: a position sensor system; a shape sensor system for determining the position, orientation, speed, velocity, pose, and/or shape of the end of the catheter device 400 and/or along one or more segments of the catheter that may constitute the catheter device 400; and/or a visualization system for capturing images from the end of the catheter device 400.

The imaging vehicle 100 may be provided with a display system 110 and a flushing system (not shown in the figure), etc. The display system 110 is used to display images or representations of the surgical site and the catheter device 400 generated by the subsystem of the sensor system 500. Real-time images of the surgical site and the catheter device 400 captured by the visualization system may also be displayed. Images of the surgical site recorded before or during surgery may also be presented using image data from imaging technologies such as computed tomography (CT), magnetic resonance imaging (MRI), optical coherence tomography (OCT), and ultrasound, etc. The preoperative or intraoperative image data may be presented as a two-dimensional, three-dimensional, or four-dimensional (e.g., time-based or rate-based) image and/or as an image from a model created from the preoperative or intraoperative image data set, and a virtual navigation image may also be displayed. In the virtual navigation image, the actual position of the catheter device 400 is registered with the preoperative image to present a virtual image of the catheter device 400 within the surgical site to the operator from the outside.

The control system 600 includes at least one memory and at least one processor. It is understood that the control system 600 can be integrated into the trolley 200 or the imaging vehicle 100, or can be independently provided. The control system 600 can support wireless communication protocols such as IEEE 802.11, IrDA, Bluetooth, HomeRF, DECT, and wireless telemetry. The control system 600 can transmit one or more signals indicating movement of the catheter instrument 400 by the power unit to move the catheter instrument 400. The catheter instrument 400 can extend to a surgical site in the body via an opening of a natural cavity of the patient or a surgical incision.

Furthermore, the control system 600 may include a mechanical control system (not shown in the figure) and an image processing system (not shown in the figure), wherein the mechanical control system is used to control the movement of the catheter device 400, and thus, may be integrated into the trolley 200. The image processing system is used for virtual navigation path planning, and thus, may be integrated into the imaging vehicle 100. Of course, the various subsystems of the control system 600 are not limited to the specific situations listed above, and may also be reasonably configured according to actual conditions. The image processing system can image the surgical site based on images of the surgical site recorded before or during surgery using the above imaging techniques. The software used in combination with manual input can also convert the recorded images into a two-dimensional or three-dimensional composite image of a part or the entire anatomical organ or segment. During the virtual navigation procedure, the sensor system 500 can be used to calculate the position of the catheter instrument 400 relative to the patient's anatomical structure, which can be used to generate an external tracking image and an internal virtual image of the patient's anatomical structure, so as to align the actual position of the catheter instrument 400 with the preoperative image, thereby presenting a virtual image of the catheter instrument 400 within the surgical site to the operator from the outside.

The inner catheter device 410 and the outer catheter device 420 have substantially the same structural composition, and each comprises a slender and flexible inner catheter 41 and an outer catheter 42, wherein the diameter of the outer catheter 42 is slightly larger than that of the inner catheter 41, so that the inner catheter 41 can pass through the outer catheter 42 and provide a certain support for the inner catheter 41, so that the inner catheter 41 can reach the target location in the patient's body, so as to facilitate operations such as tissue or cell sampling from the target location.

Certain movements of the main controller 300 may cause corresponding movements of the catheter device 400. For example, when the operator operates the direction lever of the main controller 300 to move upward or downward, the movement of the direction lever of the main controller 300 may be mapped to the corresponding pitch movement of the end of the catheter device 400; when the operator operates the direction lever of the main controller 300 to move left or right, the movement of the direction lever of the main controller 300 may be mapped to the corresponding yaw movement of the end of the catheter device 400. In this embodiment, the main controller 300 may control the end of the **catheter device 400 to move within a 360° spatial range.**

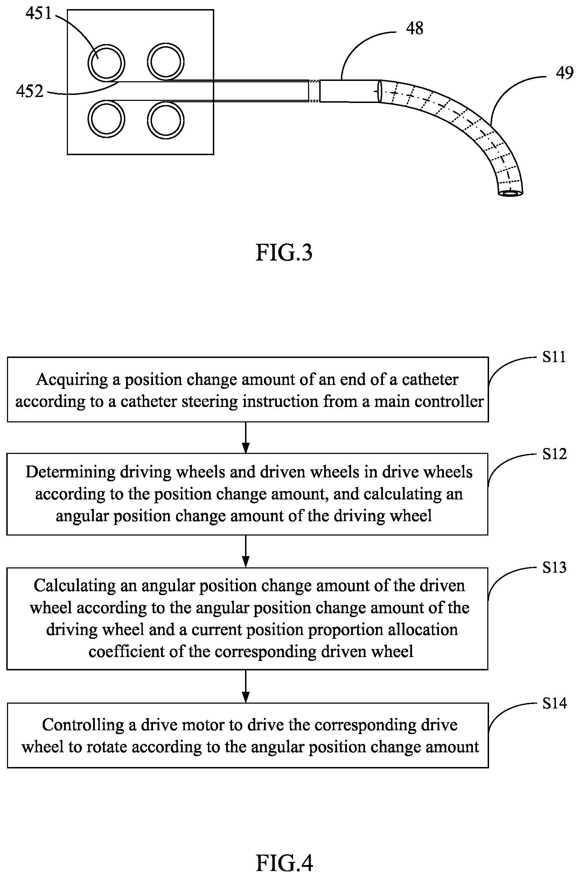

FIG. 2 and FIG. 3 illustrate a catheter instrument 400 provided in an embodiment of the present application. The catheter instrument 400 is detachably connected to the power unit 240 of the mechanical arm 230, and the catheter instrument 400 includes an instrument box 45 detachably connected to the power unit 240 and a catheter 48 connected to the instrument box 45. When the instrument box 45 is installed in the power unit 240, a driving force of the power unit 240 can be transmitted to the instrument box 45, and the catheter 48 can be moved normally. For example, under the driving force of the power unit 240, the end of the catheter 48 can be bent and turned.

The instrument box 45 includes a plurality of drive wheels 451 driven by the power unit 240 and a plurality of drive wires 452. The power unit 240 includes a plurality of drive motors 241. The drive motors 241, the drive wheels 451 and the drive wires 452 are arranged in a one-to-one correspondence. Each drive wheel 451 is detachably connected to the corresponding drive motor 241. When the instrument box 45 is installed to the power unit 240, the corresponding drive motor 241 can drive the drive wheel 451 to rotate, and the drive wheel 451 is wound with the corresponding drive wire 452. The movable part of the corresponding drive wire 452, that is, the part not wound on the drive wheel 451, extends into the catheter 48, extends along a length direction of the catheter 48 and is finally fixed to the end of the catheter.

The end in this application may also be referred to as the distal end or the head, which refers to the end away from the instrument box 45; the front end may also be referred to as the proximal end or the tail, which refers to the end close to the instrument box 45.

A portion of the catheter 48 including the end is a rotating section 49, and an end of the rotating section 49 is the end of the catheter 48. The rotating section 49 may be a joint component, which has high rigidity in the telescopic direction and low rigidity in the bending direction, and may bend under the control of the drive wire 452, thereby achieving the turning of the catheter 48. In some embodiments, the joint component may be referred to as a snake bone.

In a plane perpendicular to a length direction of the rotating section 49, the position of each drive wire 452 can be considered to be fixed, and the drive wire 452 can be extended or shortened along the length direction of the rotating section 49. The end of the drive wire 452 is fixed to the end of the rotating section 49, which does not necessarily mean that the fixed position of the end of the drive wire 452 is located in the end plane of the rotating section 49. In practical applications, in order to better protect the devices (such as endoscopes, surgical instruments, etc.) that may be carried by the catheter 48, the end of the drive wire 452 can be fixed at a position that is a short distance from the end plane of the rotating section 49 to the proximal end. In this case, if the ratio of this distance to the length of the rotating section 49 is less than a threshold value, the position where the end of the drive wire 452 is fixed can be regarded as being within the end plane of the rotating section 49 during the process of driving the end of the catheter 48 to turn.

The drive wheel 451 can rotate clockwise or counterclockwise under the drive of the corresponding drive motor 241. If the drive wheel 451 rotates in one direction, more of the corresponding drive wire 452 will be wound around the drive wheel 451, that is, part of the drive wire 452 originally belonging to the active part enters a winding state, resulting in a shortening of the length of the active part. This process can also be called pulling the drive wire 452 or pulling/winding the wire. For the convenience of description, this direction is called the positive direction. If the drive wheel 451 rotates in one direction, less of the drive wire 452 will be wound around the drive wheel 451, that is, part of the drive wire 452 originally wound around the drive wheel 451 becomes an active part, resulting in a longer length of the active part. This process can also be called wire release. For ease of description, this direction is called reverse direction. The clockwise and counterclockwise directions can be determined to be the positive direction and the counterclockwise direction to be the reverse direction according to the winding direction of the corresponding drive wire 452.

The processor of the control system 600 is configured to perform the following steps to implement the catheter bending and steering control method provided in an embodiment of the present application. As shown in FIG. 4, the method includes:

Step S11: Acquiring a position change amount of an end of a catheter according to a catheter steering instruction from a main controller.

**The position change amount may include a direction angle $\alpha$ and a bending angle $\theta$ for controlling the bending** and turning of the catheter. The user may operate at least one of the input devices such as a direction lever and a button on the main controller to input the catheter turning instruction.

Taking the direction lever as an example, the user can push the direction lever in any direction within the operation plane. This operation is converted into an electrical signal under the action of a sensor in the main controller, which is the catheter steering instruction, specifically including a first voltage and a second voltage collected on two mutually perpendicular axes of the main controller (i.e., the axes of the operation plane). The direction angle of the user's operation is calculated based on the ratio of the first voltage to the second voltage. Specifically, the inverse tangent function or inverse cotangent function of the ratio can be calculated to obtain the direction angle of the user's operation.

The arithmetic square root of the sum of the squares of the first voltage and the second voltage is calculated as a composite value of the first voltage and the second voltage. The composite value reflects the strength of the user's operation. The bending speed of the end of the catheter can be calculated based on the composite value. For example, the product of the composite value and the preset coefficient can be calculated as the bending speed. The bending angle of the user operation is obtained by calculating the integral of the bending speed and the command holding time. The command holding time refers to the duration of the same user operation.

The control interval is the interval between two adjacent steering controls of the end of the catheter. In some embodiments, there is only one catheter steering instruction in each control interval, and the sampling interval of the main controller is fixed. In this case, the integration process can be omitted, and the bending speed is directly used as the **bending angle $\theta$ operated by the user.**

In order to reduce the influence of operator's shaking, noise of main controller and the like, the direction angle and bending angle of user's operation may be calculated after smoothing and filtering the collected first voltage and the second voltage.

**The bending angle operated by the user can be directly used as the bending angle $\theta$ in the position change. In** addition, if the coordinate system of the operation plane is consistent with the coordinate system of the front plane of the catheter rotation section, that is, the rotation angle between the two is 0, then the direction angle operated by the user can **be directly used as the direction angle $\alpha$ in the position change. Otherwise, the direction angle operated by the user can** be transformed according to the mapping relationship between the two coordinate systems to obtain the direction angle $\alpha$**.**

Alternatively, in addition to the necessary angle transformation, in order to make the steering smoother and reduce jumps, the calculated direction angle and bending angle of the user operation can be divided into multiple parts to **obtain the direction angle $\alpha$ and bending angle $\theta$ in the position change. For example, if the bending angle operated** by **the user is 30°, the catheter steering instruction input by the user this time can be completed in 10 times, and the** bending **angle $\theta$ in the position change used each time is 3°.**

Step S12: determining driving wheels and driven wheels in drive wheels according to the position change amount, and calculating an angular position change amount of the driving wheel.

**To simplify the description, the direction angle $\alpha$ in the subsequent position change is referred to as the direction** angle change amount $\alpha$**, and the bending angle $\theta$ in the position change is referred to as the bending angle change** amount $\theta$**, which are not necessarily the same as the direction angle and bending angle input by the user operation.**

To facilitate the description of the posture of the rotating section, coordinate systems are established for the front plane and the end plane of the rotating section. To simplify the calculation, the focus of the coordinate system is the center of the plane. The coordinates of a certain drive wire in the front/end plane refer to the coordinates of the fixed position of the drive wire in the front/end plane, and the angle refers to the angle between the vector pointing from the origin to the coordinate of the drive wire and the positive x-axis of the front/end plane. The coordinates and angles of the same drive wire in these two coordinate systems are consistent.

When the assembled catheter is in a natural state, that is, when the end is not turned, the coordinate system of the end plane of the rotating section is generally consistent with the coordinate system of the front plane. In the process of controlling the bending/turning of the end of the catheter, it can be considered that the coordinate system of the front plane of the rotating section remains unchanged, and the coordinate system of the end plane will change under the action of the drive wire.

When the assembled catheter is in a natural state, the drive wire on each drive wheel can be maintained at a suitable tightness. If a certain drive wire is pulled, the drive wire will drive the end of the catheter to bend and turn toward its fixed position, and the bending of the end of the catheter will pull part or all of the other drive wires. If the pulling force on the drive wire caused by the bending of the end of the catheter is large enough, it will drive the corresponding drive wheel to rotate in the opposite direction for a short distance, which is generally affected by the tightness and elastic modulus of the drive wire. During the reverse rotation, since the change in pulling force is discontinuous, the jump in force will cause the corresponding drive wheel speed to jump, which may cause the wire to loosen. After rotating this distance, the drive wire is tightened. If the corresponding motor does not drive the drive wheel to rotate, the catheter will get stuck. If the pulling force on the drive wire caused by the bending of the end of the catheter is not enough to overcome the resistance and drive the corresponding drive wheel to rotate in the opposite direction, the catheter will get stuck after the drive wire is tightened to the limit determined by the system parameters (such as mechanical structure and size, material properties, etc.). The catheter is stuck, which means that the driving wheel cannot continue to reel in the line, which often results in the driving wheel being unable to rotate to the position indicated by the angular position change amount, and the end of the catheter cannot bend normally to the target position. Therefore, in order for the driving wheel to reel in the line normally, part or all of the drive wheel corresponding to the pulled drive wire need to serve as driven wheels to follow the release of the line.

Taking a pair of relatively arranged drive wheels as an example, the relative arrangement means that the line connecting the drive wires corresponding to the two drive wheels at the fixed positions at the end of the catheter passes through the center of the plane at the end of the catheter, that is, the angle difference between the drive **wires is $\pi$. When** one of the drive wheels rotates forward as the driving wheel to pull the wire, the other drive wheel needs to follow as the driven wheel to release the wire.

The number of drive wires is n, where n is an integer greater than 2, and the n drive wires divide the front plane of the catheter rotating section into n intervals in terms of angle. The drive wires corresponding to the two ends of the **interval to which the direction angle variation $\alpha$ belongs can be used as driv**ing wires, and the drive wheels connected to the driving wires is the driving wheels. Some or all of the remaining drive wheels are driven wheels. When determining **the interval to which the direction angle variation $\alpha$ belongs, the endpoints of the interval are not included.** If the **direction angle change $\alpha$ falls on a certain endpoint, that is, the angle is the same as that of a certain driv**e wire, only the drive wire can be selected as the driving wire. In some embodiments, if the number and position of the drive wires support, the number of driving wheels can be greater than 2. When the driving wheels are determined, some or all of the drive wheels other than the driving wheels can be selected as driven wheels according to the number and position allocation of the drive wires.

The following is an example of a specific method for identifying the driving wheels and the driven wheels. To simplify the description, in the following example, the serial numbers of the corresponding drive wires, drive wheels and motors are the same, but they may be different in reality; the drive wires are evenly distributed, but they may not be evenly distributed in reality.

Example 1:

In this example, n=3 and the drive wires are evenly distributed. The angles of the drive wires 1, 2, and 3 in the **front plane coordinate system of the rotating section are 0, $2\pi/3$, and $4\pi/3$ respectively.**

If the direction angle change amount **is one of 0, $2\pi/3$, $4\pi/3$, the driv**e wheel connected by the drive wire with the same angle as the direction angle change amount is the driving wheel, and the remaining two drive wheels are driven wheels.

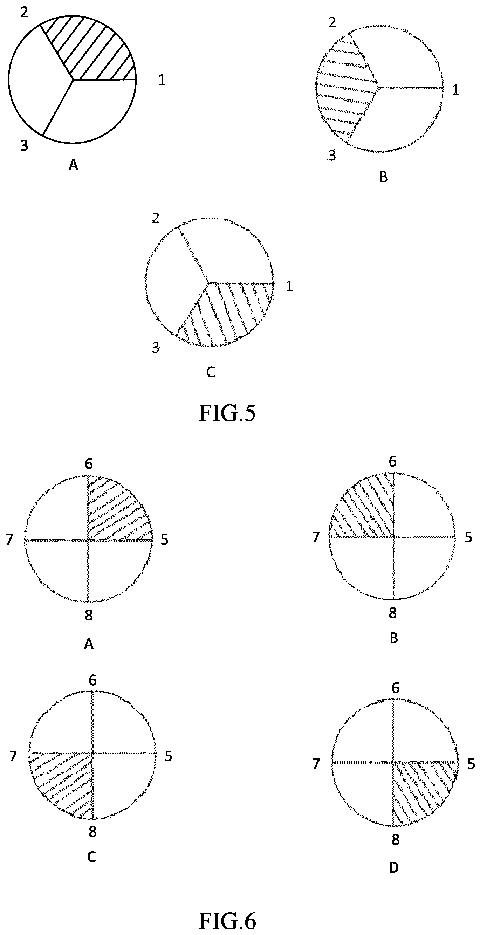

If the direction angle change amount $\alpha \in (0, \mathbf{2\pi/3)}$, that is, it falls into the shadow of part A in FIG. 5, then drive wheels 1 and 2 are driving wheels, and drive wheel 3 is the driven wheel.

If the direction angle change amount $\alpha \in \mathbf{(2\pi/3, 4\pi/3)}$, that is, it falls into the shadow of part B in FIG. 5, then drive wheels 2 and 3 are driving wheels, and drive wheel 1 is the driven wheel.

If the direction angle change amount $\alpha \in \mathbf{(4\pi/3}, 0)$, that is, it falls into the shadow of part C in FIG. 5, then drive wheels 3 and 1 are driving wheels, and drive wheel 2 is the driven wheel.

Example 2:

In this example, n=4 and the drive wires are evenly distributed. The angles of the drive wires 5, 6, 7, and 8 in the **front plane coordinate system of the rotating section are 0, $\pi/2$, $\pi$, and $3\pi/2$** respectively.

If the direction angle change amount is one of **0, $\pi/2$, $\pi$,** and $3\pi/2$, the drive wheel connected by the drive wire with the same angle as the direction angle change amount is the driving wheel, the drive wheel arranged opposite to the driving wheel is the driven wheel. For example, if the direction angle change amount **is $\pi/2$, the driv**e wheel 6 is the driving wheel, the drive wheel 8 is the driven wheel, and the drive wheels 5 and 7 are neither driving wheels nor driven wheels, that is, they do not need to rotate.

If the direction angle change amount $\alpha \in (0, \pi/2)$, that is, it falls into the shadow of part A in FIG. 6, then drive wheels 5 and 6 are driving wheels, and drive wheels 7 and 8 are the driven wheels.

If the direction angle change amount $\alpha \in \mathbf{(\pi/2, \pi)}$, that is, it falls into the shadow of part B in FIG. 6, then drive wheels 6 and 7 are driving wheels, and drive wheels 8 and 5 are the driven wheels.

If the direction angle change amount $\alpha \in (\pi, 3\pi/2)$, that is, it falls into the shadow of part C in FIG. 6, then drive wheels 7 and 8 are driving wheels, and drive wheels 5 and 6 are the driven wheels.

If the direction angle change amount $\alpha \in (3\pi/2, 0)$, that is, it falls into the shadow of part D in FIG. 6, then drive wheels 8 and 5 are driving wheels, and drive wheels 6 and 7 are the driven wheels.

By analogy, when n is an odd number greater than 4 and the drive wires are evenly distributed, if the direction angle change amount $\alpha$ **is the same as the angle of a certain drive wire, the drive wheel corresponding to the drive wire** can be selected as the driving wheel, and the drive wheels corresponding to the two drive wires whose angles are closest **to the reverse angle $\alpha \pm \pi$ of the direction angle change are selected as the driven wheels; if the reverse angle $\alpha \pm \pi$ of the** direction angle change amount is the same as the angle of a certain drive wire, the drive wheel corresponding to the drive wire can be selected as the driven wheel, and the drive wheels corresponding to the two drive wires whose angles are closest to the direction angle change amount $\alpha$ **can be selected as the driving wheels; if the direction angle change** amount $\alpha$ **and its reverse angle $\alpha \pm \pi$ are different from the angles of any driv**e wire, the drive wheels corresponding to the two drive wires whose angles are closest to the direction angle change amount $\alpha$ **can be selected as the driving wheels,** and the drive wheels corresponding to the two drive **wires whose angles are closest to the reverse angle $\alpha \pm \pi$ of the** direction angle change amount can be selected as the driven wheels. The unselected drive wheels do not need to move.

When n is an even number greater than 4 and the drive wires are evenly distributed, if the direction angle change amount $\alpha$ **is the same as the angle of a certain driv**e wire, the drive wheel corresponding to the drive wire can be selected as the driving wheel, and the drive wheel corresponding to the drive **wire with the same angle as the reverse angle $\alpha \pm \pi$** of the direction angle change amount can be selected as the driven wheel, if the direction angle change amount $\alpha$ **is** different from the angle of any drive wire, the drive wheels corresponding to the two drive wires whose angles are closest to the direction angle change amount $\alpha$ **can be selected as the driving wheels, and the driv**e wheels corresponding to the two drive **wires whose angles are closest to the reverse angles $\alpha \pm \pi$ of the direction angle change** amount can be selected as the driven wheels. The unselected drive wheels do not need to move.

In addition, there is a special case of n=2, that is, the two drive wires are arranged opposite to each other. In this case, the direction angle change amount of the drive wire controlling the rotation of the end of the catheter can actually only be the angle of one of the two drive wires. The drive wheel corresponding to the drive wire with the same direction angle change amount is the driving wheel, and the other drive wheel is the driven wheel.

Generally speaking, under the action of the drive wire, the rotating section/the end of the catheter can be bent with two degrees of freedom, and the state/posture of the rotating section/the end of the catheter can be described by two joint variables: the bending angle and the direction angle. Usually, the direction angle is used to describe the direction in which the rotating section/the end of the catheter **is bent, and its value range can be $2\pi$. The specific range can be determined according to actual needs, such as $[0, 2\pi]$, $[-\pi, \pi]$, etc. The bending angle is used to describe the degree of** bending of the rotating section/the end of the catheter. The lower limit of its value range can be 0, indicating a natural **state without bending, and the upper limit can be determined according to actual conditions, generally not exceeding $\pi$, for example, it can be $\pi/2$, $2\pi/3$, etc.**

Under the action of the position change amount, the end of the catheter changes from the current state to the target state, that is, from the current posture to the target posture. In the catheter steering control, the focus is on the transformation of the end of the catheter from the current position to the target position, and based on this, the length change of the driving wire, that is, the wire-receiving length of the driving wheel, is calculated, and then the angular position change amount of the driving wheel is calculated. The following is an example of the specific calculation process of the angular position change amount of the driving wheel with reference to the accompanying drawings.

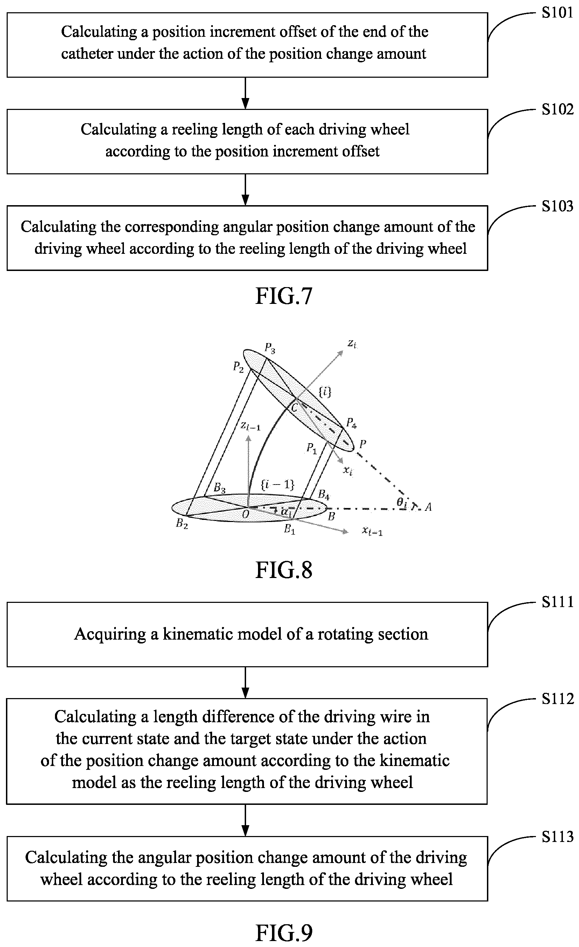

As shown in FIG. 7, in a specific embodiment of the present application, calculating the angular position change amount of the driving wheel includes the following sub-steps:

S101: calculating a position increment offset of the end of the catheter under the action of the position change amount.

Due to the structural design of the rotating section, the rotating section can be approximately treated as a circular arc during the bending and turning process. Based on this characteristic, a kinematic model can be established for the rotating section, and the position increment offset can be calculated based on the kinematic model.

For example, a kinematic model as shown in FIG. 8 can be established for the rotating section, where the length of the rotating section is L, and the rotating section is abstracted as a circle with a radius of r in the direction perpendicular to the length. The front coordinate system of the rotating section is $T_0(x_0, y_0, z_0)$, the xoy plane is the front plane and the origin is the center of the front plane. During the rotation process, it can be treated as fixed, playing a role similar to the world coordinate system. The end coordinate system of the rotating section in the current state is $T_i(x_i, y_i, z_i)$, the xoy plane is the end plane and the origin is the center of the end plane. The transformation of the end coordinate system of the rotating section in the current state relative to the front coordinate system can be described by the current **direction angle $\alpha_i$ and the current bending angle $\theta_i$. More specifically, the current bending angle $\theta_i$** is the arc angle of the arc with a length of L formed from the center of the front plane to the center of the end plane in the current state, and the **current direction angle**

$\alpha_i$ is the rotation angle of the xoy plane of the end coordinate system relative to the xoy plane of the front coordinate system in the current state. Based on this model, in the Cartesian coordinate system, the transformation matrix of the end coordinate system relative to the front coordinate system in the current state can be expressed as:

$$T_i^0(\alpha_i, \theta_i) = \begin{bmatrix} R_i^0(\alpha_i, \theta_i) & P(\alpha_i, \theta_i) \\ 0 & 1 \end{bmatrix}$$

Where $R_i^0(\alpha_i, \theta_i)$ represents the posture transformation of the end coordinate system relative to the front coordinate system in the current state; $P(\alpha_i, \theta_i)$ represents the position transformation of the end coordinate system relative to the front coordinate system in the current state, which can be represented by a vector pointing from the center of the front plane to the center of the end plane in the current state.

The sum of the current direction angle $\alpha_i$ **and the direction angle** $\alpha$ **in the position change** amount is calculated as **the target direction angle** $\alpha_{i+1}=\alpha_i+\alpha$**, and the sum of the current bending angle** $\theta_i$ **and the bending angle** $\theta$ **in the position** change amount **is calculated as the target bending angle** $\theta_{i+1}=\theta_i+\theta$**. In practical applications, if the calculated target direction angle exceeds the set value range, it can be added or subtracted by** $2\pi$ **to return to the value range;** if the calculated target bending angle is greater than the upper limit of the bending angle value range, it can be corrected to the upper limit of the bending angle value range; if the calculated target bending angle is a negative number, the target **direction angle is added or subtracted by** $\pi$ **according to the direction angle value range to change it to its opposite, and** the target bending angle is modified to its absolute value.

In the Cartesian coordinate system, the transformation matrix of the end coordinate system relative to the front coordinate system in the target state can be expressed as:

$$T_{i+1}^0(\alpha_i, \theta_i) = \begin{bmatrix} R_{i+1}^0(\alpha_{i+1}, \theta_{i+1}) & P(\alpha_{i+1}, \theta_{i+1}) \\ 0 & 1 \end{bmatrix}$$

Where $R_{i+1}^0(\alpha_{i+1}, \theta_{i+1})$ represents the posture transformation of the end coordinate system relative to the front coordinate system in the target state; $P(\alpha_{i+1}, \theta_{i+1})$ represents the position transformation of the end coordinate system relative to the front coordinate system in the target state, which can be represented by a vector pointing from the center of the front plane to the center of the end plane in the target state.

According to the position transformation in the current state and the position transformation in the target state, the position increment offset $P_i=P(\alpha_{i+1}, \theta_{i+1})-P(\alpha_i, \theta_i)$ can be calculated, which can be represented by a vector pointing from the center of the end plane in the current state to the center of the end plane in the target state.

S102: calculating a reeling length of each driving wheel according to the position increment offset.

The position increment offset describes the transformation of the end of the catheter from the current position to the target position. The drive wires can be added to the above kinematic model, and the fixed position of each drive wire is set on a circle perpendicular to the length direction. The drive wire can be treated as a straight line, an arc, a multi-section broken line, or a multi-section arc in the length direction. When discussing the contents related to the kinematic model, except for the parts specifically pointed out, the drive wire mentioned generally refers to the drive wire in the rotating section. According to the kinematic model, the mapping function between the position increment offset and the driving wheel reeling length (i.e., the shortening amount of the driving wire) can be calculated, and then the position increment offset obtained in S101 can be substituted to calculate the reeling length of each driving wheel.

The mapping function may be an analytical solution calculated according to the kinematic model, or a function obtained by processing the analytical solution by means of approximation, fitting, etc. in order to simplify calculations.

S103: calculating the corresponding angular position change amount of the driving wheel according to the reeling length of the driving wheel.

Specifically, the value of the angular position change amount can be calculated by combining the geometric parameters of the drive wheel (such as the winding radius of the drive wire) and the reeling length of the drive wheel. The rotation direction of the drive wheel (clockwise or counterclockwise) can be determined according to the winding direction of the drive wire. The angular position change amount of the driving wheel can be obtained by combining the two.

As shown in FIG. 9, in another specific embodiment of the present application, calculating the angular position change amount of the driving wheel includes the following sub-steps:

S111: acquiring a kinematic model of a rotating section.
S 112: calculating a length difference of the driving wire in the current state and the target state under the action of the position change amount according to the kinematic model as the reeling length of the driving wheel.

Still taking the kinematic model shown in FIG. 8 as an example, the number of drive wires is 4 and evenly distributed, and each drive wire is abstracted as a straight line for processing. The lower plane in FIG. 8 is the front plane, represented by i-1, B1, B2, B3, B4 are the fixed points of drive wires 1, 2, 3, 4 on the front plane, respectively, O is the center of the front plane, for the convenience of calculation, $OB_1$ is defined as the positive direction of x-axis of the front coordinate system, $OB_2$ is the positive direction of y-axis of the front coordinate system, and the z-axis of the front coordinate system is perpendicular to the front plane and points to the end plane. The upper plane in FIG. 8 is the end plane, represented by i, $P_1$, $P_2$, $P_3$, and $P_4$ are the fixed points of the drive wires 1, 2, 3, and 4 on the end plane, respectively, and C is the center of the end plane. For the convenience of calculation, $CP_1$ is defined as the positive direction of x-axis of the end coordinate system, $CP_2$ is the positive direction of y-axis of the end coordinate system, and the z-axis of the terminal coordinate system is perpendicular to the end plane and points to the side away from the front plane.

According to the model, the length of the j-th drive **wire in the current state ($\alpha_i, \theta_i$)** can be obtained:

$$l_{i,j} = \left| \overrightarrow{B_j P_j} \right| = 2\frac{L}{\theta_i}\sin\frac{\theta_i}{2} - 2r\cos\beta_j\sin\frac{\theta_i}{2}$$

$$\beta_j = \alpha_i + (j-1)\frac{\pi}{2}$$

Where j=1,2,3,4.

**Substituting the target state** ($\alpha_{i+1}, \theta_{i+1}$) into the above formula, the length of the j-th drive wire under the target **state** ($\alpha_{i+1}, \theta_{i+1}$) can be obtained:

$$l_{i+1,j} = 2\frac{L}{\theta_{i+1}}\sin\frac{\theta_{i+1}}{2} - 2r\cos\beta_j\sin\frac{\theta_{i+1}}{2}$$

$$\beta_j = \alpha_{i+1} + (j-1)\frac{\pi}{2}$$

According to the number of the driving wheel determined previously, the length change amount of the driving wire between the current state and the target state is calculated as the reeling length of the driving wheel. If the drive wheel numbered j is the driving wheel, the reeling **length** $\Delta l_j$ directly calculated according to the kinematic model is:

$$\Delta l_j = 2\frac{L}{\theta_i}\sin\frac{\theta_i}{2} + 2r\cos\left(\alpha_{i+1} + (j-1)\frac{\pi}{2}\right)\sin\frac{\theta_{i+1}}{2}$$

$$-2\frac{L}{\theta_{i+1}}\sin\frac{\theta_{i+1}}{2} - 2r\cos\left(\alpha_i + (j-1)\frac{\pi}{2}\right)\sin\frac{\theta_i}{2}$$

The above formula can be processed by approximation, fitting, etc. to simplify the calculation.

S113: calculating the angular position change amount of the driving wheel according to the reeling length of the driving wheel.

Specifically, the value of the angular position change amount can be calculated by combining the geometric parameters of the drive wheel (such as the winding radius of the drive wire) and the reeling length of the driving wheel, the rotation direction of the driving wheel (clockwise or counterclockwise) can be determined according to the winding direction of the drive wire, the angular position change amount of the driving wheel can be obtained by combining the two.

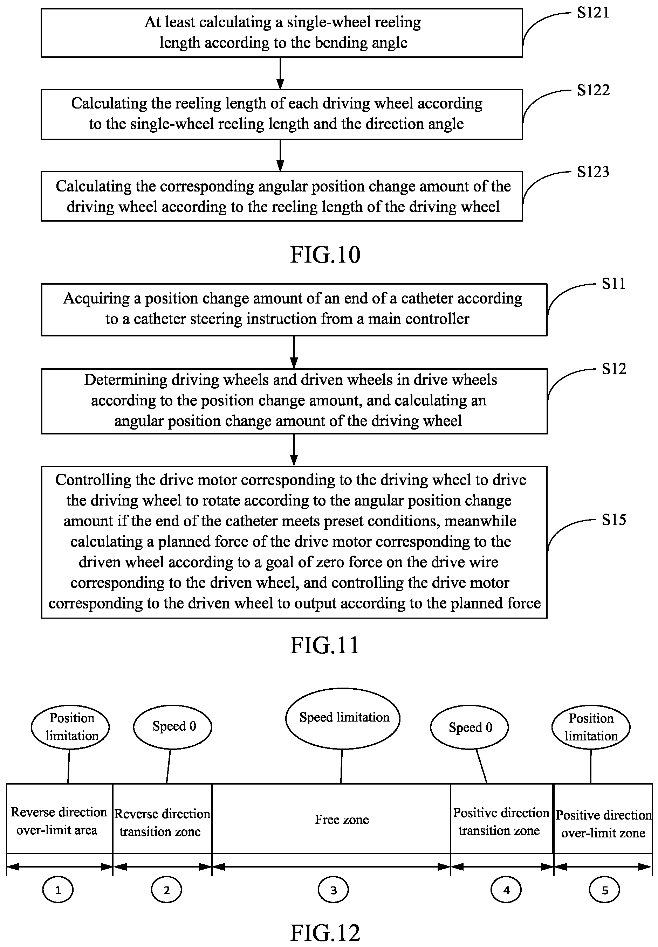

As shown in FIG. 10, in another specific embodiment of the present application, calculating the angular position change amount of the driving wheel includes the following sub-steps:

S121: at least calculating a single-wheel reeling length according to the bending angle.

In this embodiment, the calculation of the reeling length is further decomposed. If the angular position change amount is consistent with the angle of a certain drive wire, only the drive wheel corresponding to the drive wire will be selected as the driving wheel. In this case, the reeling length of the driving wheel is the single-wheel reeling length. The single-wheel reeling length is not affected by the direction angle change amount, and can be determined by the current bending angle at the end of the rotating section and the bending angle change amount.

Still taking the kinematic model shown in FIG. 8 as an example, the specific description of the kinematic model can refer to the relevant content of the above embodiment. The single-wheel reeling **length** $\Delta l$ directly calculated

according to the model is:

$$\Delta l = 2\frac{L}{\theta_i}\sin\frac{\theta_i}{2} - 2\frac{L}{\theta_{i+1}}\sin\frac{\theta_{i+1}}{2} - 2r(\sin\frac{\theta_i}{2} - \sin\frac{\theta_{i+1}}{2})$$

The above formula can be processed by approximation, fitting, etc. to simplify the calculation.

S122: calculating the reeling length of each driving wheel according to the single-wheel reeling length and the direction angle.

In the actual control process, the direction angle change amount is often different from the angle of the drive wire, and two drive wheels need to work together to pull the wire to control the end of the catheter to turn according to the direction angle change amount. The single-wheel reeling length can be mapped to the reeling length of each driving wheel according to the angle between the direction angle change amount and each drive wire.

S123: calculating the corresponding angular position change amount of the driving wheel according to the reeling length of the driving wheel.

Specifically, the value of the angular position change amount can be calculated by combining the geometric parameters of the drive wheel (such as the winding radius of the drive wire) and the reeling length of the driving wheel, the rotation direction of the driving wheel (clockwise or counterclockwise) can be determined according to the winding direction of the drive wire, the angular position change amount of the driving wheel can be obtained by combining the two.

In the example of the kinematic model given above, the drive wire is treated as a straight line. However, there is an error between the model and the actual situation, and the error will expand nonlinearly as the bending angle of the rotating section/the end of the catheter increases. In order to improve the accuracy of steering control, a more complex but more accurate kinematic model can be selected, such as treating the drive wire as a multi-section broken line/multi-section arc, treating the rotating section as a multi-section circular arc, etc. In addition, other calculation methods other than the kinematic model can be introduced as needed to modify the calculation formula of the reeling length and/or correct the results of the model calculation.

The calculation methods given above can be used one by one or in combination. For example, one of the calculation methods can be selected according to the bending angle segmentation. Experiments can be used to determine how to segment the bending angle and/or the calculation method corresponding to each segment to meet the requirements of steering control accuracy and/or real-time performance.

Step S13: calculating an angular position change amount of the driven wheel according to the angular position change amount of the driving wheel and a current position proportion allocation coefficient of the corresponding driven wheel.

The position proportion allocation coefficient is used to indicate the proportion of the reeling length of the driving wheel to the release length of the corresponding driven wheel. For a catheter equipped with n drive wheels, each drive wheel can theoretically have n-1 position proportion allocation coefficients, which are used to indicate the position proportion allocation coefficient of itself as a driven wheel when other drive wheels except itself are used as driving wheels. For the i-th drive wheel, its position proportion allocation coefficient can be expressed as a set [kij], i, j=1,...,n **and i≠j. In practical applications, according to the number of driv**e wheels and the position allocation of the corresponding drive wires, some position proportion allocation coefficients can be fixed as 0, in which case these position proportion allocation coefficients can be omitted from the set. For example, when n=4 and the drive wires are evenly distributed, the position proportion allocation coefficients between the drive wheels corresponding to two adjacent drive wires can be fixed as 0, and only the position proportion allocation coefficients between the two pairs of oppositely arranged drive wheels are retained.

If the rotating section can be treated as a rigid body, the position proportion allocation coefficient is a constant. However, the deformation of the rotating section during the movement is often not negligible, and the actual position proportion allocation coefficient changes nonlinearly. In order to more accurately describe the position proportion allocation coefficient, the current position proportion allocation coefficient is defined to represent the proportion of the reeling length of the driving wheel to the corresponding release length of the driven wheel in the current state.

If the current position proportion allocation coefficient is the proportion of the corresponding driven wheel release length to the driving wheel reeling length, the angular position change amount of the driven wheel can be the sum of the product of the angular position change amount of the driving wheel corresponding to the driven wheel and the current position proportion allocation coefficient.

The calculation function of the angular position change amount of the driven wheel can be independent or combined with the calculation function of the angular position change amount of the driving wheel. For example, if the position increment offset is used for calculation, a combined function, the angle increment allocation function, can be set for all drive wheels. The input of this function can include the current position proportion allocation coefficient, the **direction angle α in the position change** amount, and the position increment offset. According to the input, the function can determine the driving wheel and the driven wheel, calculate the angular position change amount of the driving wheel and the angular position change amount of the driven wheel, and finally output the angular position change amount of each drive wheel.

Before this step, the current position proportion allocation coefficient can be obtained. The current bending angle of the end of the catheter is input into the pre-stored mapping relationship to obtain the current position proportion allocation coefficient of the driven wheel. The output of the mapping relationship includes the current position proportion allocation coefficient, and the input includes at least the current bending angle.

Multiple groups of test samples can be collected in advance, and the samples can be processed by piecewise function, curve (such as polynomial) fitting, neural network training, etc. to obtain the mapping relationship. During the sample collection process, the driven wheel can be adjusted to a suitable position by manual control, that is, a position that meets the wire release condition, and then the angular position of the driven wheel is recorded to calculate the wire release length/angular position change amount. The wire release condition can include that the driving wheel moves to the target position this time and the driven wire wound on the driven wheel maintains a suitable tightness.

According to the structural design of the rotating section, the nonlinear change of the position proportion allocation coefficient is sensitive to the bending angle, and each group of samples includes at least the current bending angle, the bending angle change of this movement and at least one of the target bending angle, the reeling length, angular position change amount and angular position of the driving wheel, and at least one of the release length, angular position change amount and angular position of the driven wheel. Of course, the direction angle may be introduced into the independent variable of the mapping relationship, and in this case, the sample may further include at least one of the current direction angle, the direction angle change amount of this movement, and the target direction angle.

For example, when n=4 and the drive wires are evenly distributed, one drive wire can be selected and stretched individually from a natural state until the set maximum bending angle range is reached. During this process, multiple groups of samples are collected, each group of samples including the angular position of the driving wheel, the angular position of the driven wheel, and the current bending angle. For the $m^{th}$ group of samples, the difference in angular position between the driving wheel and the driven wheel in the $m-1^{th}$ group of samples can be calculated, and then the proportion of the difference in angular position of the driven wheel to the difference in angular position of the driving wheel can be calculated as the current position proportion allocation coefficient of the $m^{th}$ group of samples. The mapping relationship can be in the form of a high-order polynomial, with the input being the current bending angle and the output being the current position proportion allocation coefficient. Multiple groups of samples are used for fitting to determine the coefficients of each term in the polynomial, and the final mapping relationship is obtained for use in subsequent control.

Step S14: controlling a drive motor to drive the corresponding drive wheel to rotate according to the angular position change amount.

The angular position change amount of the corresponding drive motor may be calculated according to the angular position change amount of the drive wheel obtained in S13 based on the engagement mode between the drive wheel and the corresponding drive motor.

The drive motor can have three basic control modes: position control, speed control, and current control (also known as moment control or torque control). The position control can be chose, in which case the control amount of the drive motor is the angular position, the sum of the current angular position of the drive motor and the angular position change amount can be calculated as the target angular position and sent to the controller of the drive motor; alternatively, the angular position change amount of the drive motor may be directly sent to the controller of the drive motor. Alternatively, speed control may be selected, in which case the control amount of the drive motor is the angular speed, and the angular speed may be obtained by dividing the angular position change amount of the drive motor by the control interval and sent to the controller of the drive motor.

In addition, the drive motor generally uses three-loop control, which are the current loop, speed loop and position loop from the inside to the outside, and the output of the outer loop is the input of the adjacent loop inside. The controller of the drive motor can complete the three-loop control by itself, or it can hand over some or all of the three loops to the host computer (the processor of the control system 600 in this application) for processing. For example, when the angular position is originally used as the control amount, the position loop of the motor control can be processed by the host computer. In this case, the processor sends the angular speed processed by the position loop to the controller of the drive motor.

Through the implementation of this embodiment, the position change amount under the action of the catheter steering instruction is used instead of the target position for control, and the drive wheels are divided into driving wheels and driven wheels. Only the angular position change amount of the driving wheel is directly calculated based on the position change amount, and the angular position change amount of the driven wheel is calculated based on the angular position change amount of the driving wheel, thereby achieving decoupling of the calculation of the angular position change amount of the driven wheel and the catheter steering instruction, thereby simplifying the calculation process of the angular position change amount of each driving wheel while achieving the bending steering control of the catheter, and shortening the response time of the catheter steering instruction.

The processor of the control system 600 is further configured to execute the following steps to implement the catheter bending and steering control method provided in another embodiment of the present application. As shown in FIG. 11, after step S12, the method may further include:

Step S15: controlling the drive motor corresponding to the driving wheel to drive the driving wheel to rotate according to the angular position change amount if the end of the catheter meets preset conditions, meanwhile calculating a planned force of the drive motor corresponding to the driven wheel according to a target of zero force on the drive wire corresponding to the driven wheel, and controlling the drive motor corresponding to the driven wheel to output according to the planned force.

Based on the way in which the drive wheel is engaged with the corresponding drive motor, the angular position change amount of the corresponding drive motor can be calculated according to the angular position change amount of the driving wheel obtained in S12.

For the driven wheel, the same position control as the driving wheel can be adopted, that is, the angular position change amount of the driven wheel is calculated, and the drive motor corresponding to the driven wheel is controlled accordingly to drive the driven wheel to rotate according to the angular position change amount.

It can be seen that in the above-mentioned position control scheme of the driven wheel, the control effect is directly related to the accuracy of the position proportion allocation coefficient, and the nonlinear change of the position proportion allocation coefficient comes from the structural design of the rotating section. The larger the bending angle at the end of the catheter, that is, the farther away from the unbent state, the more obvious the nonlinear change of the position proportion allocation coefficient is.

In order to improve the control effect, in the above position control scheme, multiple sets of samples are collected in advance and processed to obtain the mapping relationship to calculate the current position proportion allocation coefficient. However, due to the inconsistency of the material properties and assembly process of the catheter, different catheters collect different samples, and even different models may be needed to build a mapping relationship. This requires that samples need to be collected for each set of catheters before use and the mapping relationship of the catheter needs to be processed, which takes a lot of time. In addition, in areas where the end of the catheter is more curved, if not enough samples are collected or the selected model is inappropriate, there may be a large error in the current position proportion allocation coefficient calculated using the mapping relationship, causing the catheter to get stuck or the driven wheel to loosen during the bending and turning process, affecting the control effect.

In order to at least partially solve the above problems, at least when the end of the catheter meets the preset conditions, the force mode is used to control the drive motor corresponding to the driven wheel. The control target is to make the drive wire corresponding to the driven wheel zero-force, that is, the drive wire corresponding to the driven wheel can move in accordance with the external force (here refers to the tension caused by the bending of the catheter) as if it is in an environment with zero force. This control method can also be called zero-force control of the drive wire corresponding to the driven wheel.

The preset conditions include that at least one of the current bending angle and the target bending angle of the end of the catheter is within a preset range. Specifically, the preset conditions may be that the current bending angle is within the preset range, or the target bending angle is within the preset range, or either the current bending angle and the target bending angle is within the preset range, or both the current bending angle and the target bending angle are within the preset range.

If the end of the catheter does not meet the preset conditions, the force mode can still be used to perform zero-force control on the drive wire corresponding to the driven wheel, or the position control can be used in the same way as the driving wheel. If the force mode is used, the process of collecting samples and building a mapping relationship can be omitted. If position control is used, since the nonlinear change of the position proportion allocation coefficient outside the preset range is less obvious, fewer samples and simple models can be used to build a sufficiently accurate mapping relationship, and even the change of the position proportion allocation coefficient can be treated as linear.

Since the position control of the driven wheel is less effective in the area where the end of the catheter is more curved, the preset range generally includes a range away from the initial value of the bending angle (i.e., the bending angle in the unbent state). Specifically, the boundary of the preset range may include a maximum bending angle threshold. In addition to the maximum bending angle threshold, another boundary of the preset range may be called a bending angle transition threshold. The maximum bending angle threshold of the end of the catheter is $T_m$, and the bending angle transition threshold is $T_g$, then the preset range can be expressed as $[T_g, T_m]$.

The value range of the bending angle transition threshold $T_g$ is $[0, T_m)$. The value of the bending angle transition threshold $T_g$ can be set according to needs. For example, the change of the position proportion allocation coefficient outside the preset range can be regarded as linear as the goal. The bending angle transition threshold $T_g$ is estimated according to system parameters (such as mechanical structure and size, material properties, etc.) or samples are collected to fit the mapping relationship between the bending angle and the position proportion allocation coefficient to determine the bending angle transition threshold $T_g$.

In order to achieve the goal of zero force on the drive wire corresponding to the driven wheel, the force on the drive wire corresponding to the driven wheel can be obtained in real time during the process of controlling the rotation of the driving wheel, and the planned force of the drive motor corresponding to the driven wheel can be set accordingly. For example, the planned force can be the same as the force on the drive wire corresponding to the driven wheel, so that the driven wheel can follow the driving wheel to release the wire. For ease of description, in this application, each force has two attributes: direction and magnitude, where the direction is simplified to positive direction and reverse direction. The positive direction can be the direction pointing away from the end of the catheter, and the positive force plays a role in driving the wire to be reeling or hindering the wire to be released; the reverse direction can be the direction pointing to the end of the catheter, and the reverse force plays a role in driving the wire to be released or hindering the wire to be reeling. Of course, it can also be the other way around, the positive direction is the direction pointing to the end of the catheter, and the positive force plays a role in driving the wire to be released or hindering the wire to be reeling; the reverse direction can be the direction pointing away from the end of the catheter, and the reverse force plays a role in driving the wire to be reeling or hindering the wire to be released. The following is an example in which the positive direction is the direction pointing away from the end of the catheter and the reverse direction is the direction pointing to the end of the catheter.

In some embodiments, a force/torque sensor may be provided on the drive wheel and/or the drive wire, and the force on the drive wire corresponding to the driven wheel, i.e., the feedback force, may be directly or indirectly obtained according to the feedback value of the force/torque sensor provided on the driven wheel and/or the drive wire corresponding to the driven wheel. Then, the planned force may be set according to the feedback force of the drive wire corresponding to the driven wheel, for example, the planned force may be set equal to the feedback force.

If no force/torque sensor is installed on the drive wheel and the drive wire, a position sensor is needed to indirectly obtain the force on the drive wire corresponding to the driven wheel. The position sensor can be installed on the drive motor or on the drive wheel/drive wire, and the object directly measured can be the angular position or the linear position. For example, the position sensor can be the encoder of the drive motor.

During the wire-releasing process, the drive wire can be regarded as a spring-damper system. According to the model of the spring-damper system, the resistance exerted on the drive wire is the sum of the spring force and the damping force. The magnitude of the spring force is proportional to the position of the drive wire, and its direction is opposite to that of the position. The position of the drive wire can be calculated using the position feedback from the position sensor. The magnitude of the damping force is proportional to the speed, and its direction is opposite to that of the speed. The speed of the drive wire can be obtained by calculating the differential of the position of the drive wire with respect to time.

The premise of using the position sensor to correctly obtain the force on the drive wire corresponding to the driven wheel is that the drive wire can rotate under the action of external force (the force output by the drive motor and/or the tension caused by the bending of the end of the catheter). In the initial stage of controlling the motor corresponding to the driving wheel to drive the driving wheel to rotate, the end of the catheter begins to bend and turn under the action of the driving wheel to reel in, exerting tension on the drive wire corresponding to the driven wheel, and the actual force on the drive wire corresponding to the driven wheel changes. If the pulling force is not enough to overcome the resistance, the driven wheel will not rotate, the position and speed obtained from the sensor will remain unchanged, and the calculated force on the drive wire will remain unchanged, which is inconsistent with the actual situation. The rotation of the driven wheel cannot be accurately controlled, which may still cause the catheter to get stuck.

In order to at least partially solve the above problem, in the initial stage of driving the driving wheel to rotate, an initial force can be given to the drive wire to help overcome the resistance, that is, the planned force is set to the initial value. The size of the initial value can be measured experimentally or estimated based on system parameters (such as mechanical structure and size, material properties, etc.), and the direction is reverse. Under the action of the initial value, the driven wheel will directly start to rotate and release the wire, or will not rotate temporarily, and then start to rotate and release the wire under the action of the tension brought by the end of the catheter.

The initial values corresponding to different drive wheels may be the same or different. For a certain drive wheel, the initial value may be a single fixed value; or the preset range may be further divided into at least two parts, each part corresponding to an initial value; or a function for calculating the initial value, in which the independent variables including the bending angle are obtained in advance through experiments/modeling, etc., is used to calculate the initial value, and then the function is used to calculate the initial value. Generally speaking, a suitable initial value should be sufficient to assist the driven wheel to start rotating before the corresponding drive wire is tightened by the end of the catheter to the limit determined by system parameters (such as mechanical structure and size, material properties, etc.).

Subsequently, in the process of driving the driving wheel to rotate, the planned force is equal to the sum of the initial value, the damping force and the spring force. The change in tension brought by the end of the catheter will affect the speed and position of the driving wheel, which in turn affects the damping force and the spring force, and thus affects the calculated planned force.

Except for the initial stage, the calculation formula of planned force can be expressed as:

$$F_{prf} = F_0 + F_v + F_p$$

Where $F_{prf}$ is the planned force, $F_0$ is the initial value, $F_v$ is the damping force, and $F_p$ is the spring force.

For example, the position sensor is an encoder installed on the drive motor, which can provide feedback on the current angular position of the motor. The position of the drive wheel can be calculated based on the size information and assembly relationship between the motor and the drive wheel. Alternatively, an encoder is provided on the drive wheel, and the position of the drive wheel can be directly obtained from the feedback signal of the encoder. The speed of the drive wheel can be obtained by calculating the differential of the position of the drive wheel with respect to the speed. For example, the current position of the drive wheel is subtracted from the previous position to obtain the difference between the two, and then the difference is divided by the time interval between the two encoder feedback moments to obtain the current speed of the drive wheel.

The damping force is calculated based on the speed of the driven wheel. Specifically, the magnitude of the damping force is positively correlated with the speed. If the spring-damper system model is strictly followed, the magnitude of the damping force is proportional to the speed, and the proportion coefficient can be called the damping coefficient. However, in actual applications, the calculation formula of the damping force can be adjusted based on the model for practical needs. The damping force and speed after adjustment may not always maintain a proportional relationship. But in general, as the current speed increases, the damping force increases or remains unchanged. This relationship is defined as a positive correlation between the damping force and the speed.

The spring force is calculated based on the position of the driven wheel. Specifically, the magnitude of the spring force is positively correlated with the position. If the spring-damper system model is strictly followed, the magnitude of the spring force is proportional to the position, and the proportion coefficient can be called the spring coefficient. However, in actual applications, the calculation formula of the spring force can be adjusted based on the model for practical needs. After the adjustment, the spring force and position may not always maintain a proportional relationship. But in general, as the current position increases, the spring force increases or remains unchanged, and this relationship is defined as a positive correlation between the spring force and the position.

Optionally, in order to make the control in the force mode more stable, a maximum value may be set for the damping force and/or spring force. If the calculated damping force and/or spring force is greater than the maximum value, it may be corrected to the maximum value. If the damping force and the spring force are both set to the maximum value, the magnitudes of the two may be the same or different.

When the force mode is used, the calculated control quantity of the drive motor is the force. If the motor loses control during the control process, for example, the position and/or exceeds the allowable range, the planned force calculated using the above formula may not be able to resolve the loss of control in a timely manner, which may cause the motor to be out of control for a long time, causing the motor to malfunction or even be damaged. In order to promptly address possible loss of control of the drive motor, position and/or speed limitations can be added to the force control.

Optionally, if the position of the driven wheel belongs to the over-limit area, the reverse maximum force corresponding to the over-limit area is used as the planned force to control the driven wheel to quickly return to the position within the allowable range. The over-limit area refers to the area of the drive wheel outside the restricted area of the joint space, and the restricted area refers to the area where the drive wheel can move freely. The over-limit area includes two sub-areas, the positive direction over-limit area and the reverse direction over-limit area, wherein the positive direction over-limit area is closer to the positive direction boundary of the restricted area, and the reverse direction over-limit area is closer to the reverse direction boundary of the restricted area. The direction of the reverse direction maximum force corresponding to the over-limit zone points from the over-limit zone to the restricted area, and the magnitude is the maximum value allowed by the system parameters (such as the parameters of the drive motor). For example, if the position belongs to the positive direction over-limit zone, the planned force is the reverse direction maximum force that the drive motor can output; if the position belongs to the reverse direction over-limit zone, the planned force is the positive direction maximum force that the drive motor can output. The positive direction and reverse direction here can be defined according to reeling and releasing, for example, the positive direction corresponds to reeling and the reverse direction corresponds to releasing, or the positive and reverse directions are defined following the rotation direction of the drive wheel, for example, the positive direction corresponds to clockwise and the reverse direction corresponds to counterclockwise.

Specifically, the catheter can be pulled to the boundary of the bending angle range at multiple direction angles in advance and the corresponding positions of each drive wheel can be recorded. These direction angles generally include the direction angles corresponding to when a single drive wheel acts as an driving wheel/driven wheel. During this process, manual adjustment or introduction of force/torque sensors can be used to maintain each drive wire in a suitable tightness state. Then, all recorded positions are counted to obtain the maximum motion range of each drive wheel. The maximum motion range can be directly used as the restricted area, or it can be appropriately adjusted (for example, appropriately expanded or appropriately reduced) as the restricted area.

Since the drive wheel generally has the largest position change when used alone as an driving wheel/driven wheel, the process of determining the maximum range of motion of the drive wheel can be simplified accordingly.

Specifically, for a drive **wire with an angle of γ in the front/end plane, the catheter can be controlled to bend to the position of (y, $\theta_t$), where** $\theta_t$ represents the maximum value of the bending angle range, and the position of the drive wheel corresponding to the drive wire at this time is recorded as its positive direction maximum value. In addition, the **catheter is controlled to bend to the position of ($\gamma+\pi$, $\theta_t$)**, and the position of the drive wheel corresponding to the drive wire at this time is recorded as its reverse direction maximum value. By performing the above process for each drive wire, the maximum motion range of each drive wheel can be obtained. In addition, if there is a pair of drive wheels arranged opposite to each other, when the position of one drive wheel reaches the maximum value in the positive direction, the position of the other drive wheel reaches the maximum value in the negative direction, and the maximum motion range of a pair of drive wheels arranged opposite to each other can be obtained by bending to two positions.

Specifically, if the speed of the driven wheel exceeds the speed limit range, the planned force is adjusted to control the speed of the driven wheel to return to the speed limit range. The planned force can be adjusted so that the direction of the force output by the drive motor immediately or quickly changes to the opposite direction of the speed, thereby reducing the speed of the driven wheel and returning the speed of the driven wheel to the speed limit range.

In addition, the restricted area of the drive wheel in the joint space can be further divided into a free zone and a transition zone, and the transition zone is located between the free zone and the over-limit zone. The control strategy in the transition zone can be the same as that in the free zone, or it can be different.

The specific control strategy is illustrated below with reference to FIG. 12, the joint space of the drive wheel is divided into ① reverse direction over-limit zone, ② reverse direction transition zone, ③ free zone, ④ positive direction transition zone, and ⑤ positive direction over-limit zone. If the current position of the driven wheel is in ① reverse direction over-limit zone, the planned force is the maximum positive direction force. If the current position of the driven wheel is in ② reverse direction transition zone, the planned force is calculated according to the target speed of 0, so that the drive wheel stops as much as possible in the reverse direction transition zone without entering the reverse direction over-limit zone. If the current position of the driven wheel is in the ③ free zone, it is determined whether the current speed exceeds the speed limit range. If it exceeds, the planned force is calculated according to the target of returning the speed to the speed limit range. Otherwise, the conventional formula is used to calculate the planned force to control the driven wheel to follow the wire reeling of the driving wheel and release the wire. If the current position of the driven wheel is in the ④ positive direction transition zone, the planned force is calculated according to the target speed of 0, so that the drive wheel stops in the positive direction transition zone as much as possible without entering the positive direction over-limit zone. If the current position of the driven wheel is in the ⑤ positive direction over-limit zone, the planned force is the maximum reverse direction force.

Under normal conditions, the planned force adopts the conventional calculation formula, that is, $F_{prf}=F_0+F_v+F_p$. Under unconventional conditions, there are new control targets, such as speed is 0 or speed returns to the speed limit range or leaves the over-limit area. At this time, the planned force can be set to the preset value, or a different planned force calculation formula can be used than under normal conditions. The direct expression of the calculation formula under the unconventional state may be different from the conventional formula, for example, by introducing new variables; or the calculation formula under the unconventional state is still $F_{prf}=F_0+F_v+F_p$, but the calculation formula of the damping force and/or spring force is different from that under the conventional state to achieve a new control target.

In addition, in order to make the control of the driven wheel smoother and reduce jumps, the calculated planned force can be segmented, smoothed, filtered, or at least one other process before being sent to the drive motor corresponding to the driven wheel.

Since there is no force in the basic control mode of the drive motor, that is, the controller of the drive motor cannot directly control the drive motor according to the force, it is necessary to convert the planned force into a control quantity that the drive motor can handle and then send it to the controller of the drive motor.

For example, the planned force can be converted into the current for controlling the drive motor according to the following formula:

$$\text{fCurrent}= F_{prf} *\text{fAxisRadius}/(\text{fDecRatio}*\text{fTrqCon})$$

Where fCurrent is the control current, $F_{prf}$ is the planned force after necessary processing, fAxisRadius is the radius of the drive wheel, fTrqCon is the current torque constant of the motor, and fDecRatio is the motor reduction ratio, that is, the transmission ratio between the drive wheel and the corresponding drive motor. Since it can be considered that the torque of the drive motor is proportional to the control current, the control torque can be obtained by calculating the product of the control current and the corresponding coefficient.

Since the planned force affects the acceleration of the drive wheel rotation, the integral of the acceleration is the speed, and the integral of the speed is the position. The target speed/position of the driven wheel can be calculated based on the planned force and the current speed/position of the driven wheel as the control amount of the corresponding drive motor.

Through the implementation of this embodiment, the position change amount under the action of the catheter steering instruction is used instead of the target position for control, and the drive wheels are divided into driving wheels and driven wheels. The angular position change amount of the driving wheel is directly calculated according to the position change amount. If the end of the catheter meets the preset conditions, in the process of controlling the driving wheel according to the angular position change amount, the driven wheel is controlled to follow the rotation according to the goal of zero force on the drive wire corresponding to the driven wheel, which is more suitable for the nonlinear changes of the catheter end in the rotation process, realizes the bending steering of the catheter, and at the same time reduces the abnormal tightness of the drive wire corresponding to the driven wheel.

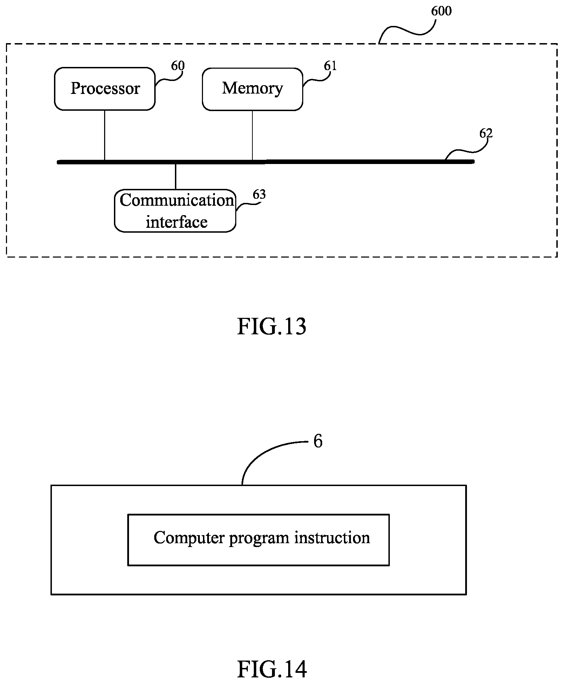

The embodiment of the present application also provides a control system for a catheter system. Please refer to FIG. 13, which illustrates a schematic diagram of the structure of the control system of the catheter system provided by an embodiment of the present application. As shown in FIG. 13, the control system 600 includes: a processor 60, a memory 61, a bus 62 and a communication interface 63, the processor 60, the communication interface 63 and the memory 61 are connected through the bus 62; the memory 61 stores computer program instructions that can be executed by the processor 60, and the processor 60 executes the catheter bending and steering control method provided in any of the aforementioned embodiments of the present application when executing the computer program instructions.

The memory 61 may include a high-speed random access memory (RAM), and may also include a non-volatile memory, such as at least one disk memory. The communication connection between the device network element and at least one other network element is realized through at least one communication interface 63 (which may be wired or wireless), and the Internet, wide area network, local area network, metropolitan area network, etc. may be used.

The bus 62 may be an ISA bus, a PCI bus, or an EISA bus, etc. The bus may be divided into an address bus, a data bus, a control bus, etc. The memory 61 is used to store a program, and the processor 60 executes the program after receiving an execution instruction. The catheter bending and steering control method disclosed in any implementation of the embodiment of the present application may be applied to the processor 60, or implemented by the processor 60.

The processor 60 may be an integrated circuit chip with signal processing capabilities. In the implementation process, each step of the above method can be completed by the hardware integrated logic circuit in the processor 60 or the instructions in the form of software. The above processor 60 can be a general-purpose processor, including a central processing unit (CPU), a network processor (NP), etc.; it can also be a digital signal processor (DSP), an application-specific integrated circuit (ASIC), a field-programmable gate array (FPGA) or other programmable logic devices, discrete gates or transistor logic devices, and discrete hardware components. The methods, steps and logic diagrams disclosed in the embodiments of the present application can be implemented or executed. The general processor can be a microprocessor or the processor can also be any conventional processor, etc. The steps of the method disclosed in the embodiments of the present application can be directly embodied as a hardware decoding processor for execution, or a combination of hardware and software modules in the decoding processor for execution. The software module can be located in a mature storage medium in the field such as a random access memory, a flash memory, a read-only memory, a programmable read-only memory or an electrically erasable programmable memory, a register, etc. The storage medium is located in the memory 61, and the processor 60 reads the information in the memory 61 and completes the steps of the above method in combination with its hardware.

The electronic device provided in the embodiment of the present application and the catheter bending and steering control method provided in the embodiment of the present application are based on the same inventive concept and have the same beneficial effects as the methods adopted, operated or implemented therein.

An embodiment of the present application also provides a computer-readable storage medium corresponding to the catheter bending and steering control method provided in the aforementioned embodiment. Please refer to FIG. 14, which shows a computer-readable storage medium 6 on which computer program instructions are stored. When the computer program instructions are executed by a processor, the catheter bending and steering control method provided in any of the aforementioned embodiments will be implemented.

It should be noted that examples of the computer-readable storage medium may include, but are not limited to, optical disks, phase change memory (PRAM), static random access memory (SRAM), dynamic random access memory (DRAM), other types of random access memory (RAM), read-only memory (ROM), electrically erasable programmable read-only memory (EEPROM), flash memory or other optical or magnetic storage media, which are not listed here one by one.

The computer-readable storage medium provided in the above-mentioned embodiment of the present application and the catheter bending and steering control method provided in the embodiment of the present application are based on the same inventive concept and have the same beneficial effects as the method adopted, run or implemented by the application program stored therein.

It should be noted that:

In the description provided herein, a large number of specific details are described. However, it is understood that the embodiments of the present application can be practiced without these specific details. In some instances, well-known structures and technologies are not shown in detail so as not to obscure the understanding of this description.

Similarly, it should be understood that in order to streamline the present application and aid in understanding one or more of the various inventive aspects, in the above description of the exemplary embodiments of the present application, the various features of the present application are sometimes grouped together into a single embodiment, figure, or description thereof. However, this disclosed method should not be interpreted as reflecting the following schematic diagram: the claimed application requires more features than those explicitly recited in each claim. Rather, as the following claims reflect, inventive aspects lie in less than all features of a single foregoing disclosed embodiment. The claims following the Detailed Description are thus hereby expressly incorporated into this Detailed Description, with each claim standing on its own as a separate embodiment of this application.

In addition, those skilled in the art will appreciate that, although some embodiments described herein include certain features included in other embodiments but not other features, the combination of features of different embodiments is meant to be within the scope of the present application and form different embodiments. For example, in the claims below, any one of the claimed embodiments may be used in any combination.

The above is only a preferred specific implementation of the present application, but the protection scope of the present application is not limited thereto. Any changes or substitutions that can be easily thought of by a person skilled in the art within the technical scope disclosed in the present application should be included in the protection scope of the present application. Therefore, the protection scope of the present application shall be based on the protection scope of the claims.

**Claims**

1. A catheter system, **characterized in that**, the catheter system comprising a mechanical arm, a catheter instrument engaged with a power unit of the mechanical arm, a main controller and a processor communicatively connected to the mechanical arm, the catheter instrument comprising an instrument box configured to be engaged with the power unit and a catheter connected to the instrument box, the instrument box comprising drive wheels configured to be driven by the power unit and drive wires having one end wound around the drive wheels and the other end extending along the catheter and fixed to an end of the catheter, the power unit comprising a plurality of drive motors, the drive motors, the drive wheels and the drive wires correspond one to one, and the processor is configured to perform the following steps:

    acquiring a position change amount of the end of the catheter according to a catheter steering instruction from the main controller, the position change amount comprising a direction angle and a bending angle;
    determining the driving wheel and the driven wheel in the drive wheels according to the position change amount, and calculating an angular position change amount of the driving wheel;
    calculating an angular position change amount of the driven wheel according to the angular position change amount of the driving wheel and a current position proportion allocation coefficient of the corresponding driven wheel, the current position proportion allocation coefficient configured to indicate a proportion of a reeling length of the driving wheel to a corresponding release length of the driven wheel in a current state;
    controlling the drive motor to drive the corresponding drive wheel to rotate according to the angular position change amount.

2. The catheter system according to claim 1, wherein a quantity of the drive wires is n, where n is an integer greater than 2, the n drive wires divide a front plane of a rotating section of the catheter into n intervals in terms of angle, the drive wire corresponding to two ends of the interval to which the direction angle belongs is a driving wire, and the drive wheel connected to the driving wire is the driving wheel, and some or all of the remaining drive wheels are the driven wheels.

3. The catheter system according to claim 2, wherein the processor is configured to perform the following specific steps:

    calculating a position increment offset of the end of the catheter under the action of the position change amount;
    calculating a reeling length of each driving wheel according to the position increment offset;
    calculating the corresponding angular position change amount of the driving wheel according to the reeling length of the driving wheel; or
    acquiring a kinematic model of the rotating section;
    calculating a length difference of the driving wire in the current state and a target state under the action of the position change amount according to the kinematic model as the reeling length of the driving wheel;
    calculating the angular position change amount of the driving wheel according to the reeling length of the driving wheel; or

at least calculating a single-wheel reeling length according to the bending angle;

calculating the reeling length of each driving wheel according to the single-wheel reeling length and the direction angle;

calculating the corresponding angular position change amount of the driving wheel according to the reeling length of the driving wheel.

4. The catheter system according to claim 1, wherein the processor is configured to perform the following steps before calculating the angular position change amount of the driven wheel according to the angular position change amount of the driving wheel and the current position proportion allocation coefficient of the corresponding driven wheel:

inputting a current bending angle of the end of the catheter into a pre-stored mapping relationship to obtain the current position proportion allocation coefficient of the driven wheel, wherein the input of the mapping relationship at least comprises the current bending angle.

5. The catheter system according to claim 1, wherein the angular position change amount of the driven wheel is a sum of a product of the angular position change amount of the driving wheel corresponding to the driven wheel and the current position proportion allocation coefficient.

6. The catheter system according to claim 1, wherein the catheter steering instruction comprises a first voltage and a second voltage collected on two mutually perpendicular axes of the main controller, the direction angle is calculated based on a ratio of the first voltage to the second voltage, the bending angle is obtained by calculating an integral of a bending speed with respect to a command holding time, and the bending speed is calculated based on a composite value of the first voltage and the second voltage.

7. The catheter system according to claim 1, wherein the processor is also configured to perform the following steps after determining the driving wheel and the driven wheel in the drive wheels according to the position change amount, and calculating the angular position change amount of the driving wheel:

controlling the drive motor corresponding to the driving wheel to drive the driving wheel to rotate according to the angular position change if the end of the catheter meets preset conditions, meanwhile calculating a planned force of the drive motor corresponding to the driven wheel according to a target of zero force on the drive wire corresponding to the driven wheel, and controlling the drive motor corresponding to the driven wheel to output according to the planned force, the preset conditions comprise that at least one of the current bending angle and the target bending angle of the end of the catheter belongs to a preset range, and the target bending angle is a sum of the current bending angle and the bending angle in the position change amount.

8. The catheter system according to claim 7, wherein in an initial stage of driving the driving wheel to rotate, the planned force is set to an initial value; subsequently, in the process of driving the driving wheel to rotate, the planned force is a sum of the initial value, a damping force and a spring force, where the damping force is calculated based on a speed of the driven wheel, and the spring force is calculated based on a position of the driven wheel.

9. The catheter system according to claim 8, wherein a magnitude of the damping force is positively correlated with the speed; a magnitude of the spring force is positively correlated with the position.

10. The catheter system according to claim 7, wherein the planned force is obtained according to a feedback force of the drive wire corresponding to the driven wheel.

11. The catheter system according to claim 7, wherein the processor is configured to perform the following steps: using a reverse direction maximum force corresponding to the over-limit zone as the planned force if the position of the driven wheel belongs to an over-limit zone.

12. The catheter system according to claim 7, wherein the processor is configured to perform the following steps: adjusting the planned force to control the speed of the driven wheel to return to a speed limit range if the speed of the driven wheel exceeds the speed limit range.

13. The catheter system according to claim 7, wherein the processor is configured to perform the following steps: calculating the angular position change amount of the driven wheel according to the angular position change amount of the driving wheel and the current position proportion allocation coefficient of the corresponding driven wheel if the end of the catheter does not meet the preset conditions, and controlling the drive motor corresponding to the driven wheel to drive the driven wheel to rotate according to the angular position change amount, the current position proportion allocation coefficient is configured to represent the proportion of the reeling length of the driving wheel to the corresponding release length of the driven wheel in the current state.

14. The catheter system according to claim 7, wherein boundaries of the preset range comprise a maximum bending angle threshold.

15. A steering control method of an end of a catheter, **characterized in that**, comprising:

acquiring a position change amount of the end of the catheter according to a catheter steering instruction from a main controller, the position change amount comprising a direction angle and a bending angle;
determining driving wheel and driven wheel in drive wheels according to the position change amount, and calculating an angular position change amount of the driving wheel, the drive wheels, drive motors, and drive wires correspond one to one, the drive wheels wound with corresponding drive wires and driven by the corresponding drive motors, the other end of the drive wire extends along the catheter and is fixed to the end of the catheter,
calculating an angular position change amount of the driven wheel according to the angular position change amount of the driving wheel and a current position proportion allocation coefficient of the corresponding driven wheel, the current position proportion allocation coefficient configured to indicate a proportion of a reeling length of the driving wheel to a corresponding release length of the driven wheel in a current state;
controlling the drive motor to drive the corresponding drive wheel to rotate according to the angular position change amount.

16. A computer-readable storage medium stores computer program instructions, wherein the computer program instructions are configured to be loaded and executed by a processor to implement the steps of the method according to claim 15.

1000

FIG.1

FIG.2

451

452

48

49

**FIG.3**

Acquiring a position change amount of an end of a catheter according to a catheter steering instruction from a main controller — S11

Determining driving wheels and driven wheels in drive wheels according to the position change amount, and calculating an angular position change amount of the driving wheel — S12

Calculating an angular position change amount of the driven wheel according to the angular position change amount of the driving wheel and a current position proportion allocation coefficient of the corresponding driven wheel — S13

Controlling a drive motor to drive the corresponding drive wheel to rotate according to the angular position change amount — S14

**FIG.4**

FIG.5

FIG.6

| | |
|---|---|
| Calculating a position increment offset of the end of the catheter under the action of the position change amount | S101 |

↓

| | |
|---|---|
| Calculating a reeling length of each driving wheel according to the position increment offset | S102 |

↓

| | |
|---|---|
| Calculating the corresponding angular position change amount of the driving wheel according to the reeling length of the driving wheel | S103 |

FIG.7

FIG.8

| | |
|---|---|
| Acquiring a kinematic model of a rotating section | S111 |

↓

| | |
|---|---|
| Calculating a length difference of the driving wire in the current state and the target state under the action of the position change amount according to the kinematic model as the reeling length of the driving wheel | S112 |

↓

| | |
|---|---|
| Calculating the angular position change amount of the driving wheel according to the reeling length of the driving wheel | S113 |

FIG.9

| At least calculating a single-wheel reeling length according to the bending angle | S121 |

↓

| Calculating the reeling length of each driving wheel according to the single-wheel reeling length and the direction angle | S122 |

↓

| Calculating the corresponding angular position change amount of the driving wheel according to the reeling length of the driving wheel | S123 |

FIG.10

| Acquiring a position change amount of an end of a catheter according to a catheter steering instruction from a main controller | S11 |

↓

| Determining driving wheels and driven wheels in drive wheels according to the position change amount, and calculating an angular position change amount of the driving wheel | S12 |

↓

| Controlling the drive motor corresponding to the driving wheel to drive the driving wheel to rotate according to the angular position change amount if the end of the catheter meets preset conditions, meanwhile calculating a planned force of the drive motor corresponding to the driven wheel according to a goal of zero force on the drive wire corresponding to the driven wheel, and controlling the drive motor corresponding to the driven wheel to output according to the planned force | S15 |

FIG.11

| Position limitation | Speed 0 | Speed limitation | Speed 0 | Position limitation |

| Reverse direction over-limit area | Reverse direction transition zone | Free zone | Positive direction transition zone | Positive direction over-limit zone |

1  2  3  4  5

FIG.12

600

Processor  60

Memory  61

62

Communication interface  63

FIG.13

6

Computer program instruction

FIG.14

## INTERNATIONAL SEARCH REPORT

| International application No. |
| --- |
| **PCT/CN2023/130041** |

| | |
| --- | --- |
| **A.** **CLASSIFICATION OF SUBJECT MATTER** | |
| A61M25/01(2006.01)i; A61B34/30(2016.01)i; A61B34/10(2016.01)i | |
| According to International Patent Classification (IPC) or to both national classification and IPC | |

| **B.** **FIELDS SEARCHED** |
| --- |
| Minimum documentation searched (classification system followed by classification symbols) |
| IPC: A61M, A61B |
| Documentation searched other than minimum documentation to the extent that such documents are included in the fields searched |
| Electronic data base consulted during the international search (name of data base and, where practicable, search terms used) |
| CNKI, CNABS, CNTXT, ENTXT, DWPI: 精锋医疗科技, 罗兴桂, 肖凡, 高元倩, 导丝, 导管, 弯曲, 偏转, 转向, 角, 方向, 变化, 比例, 驱动, 轮, 主动, 从动, 偏移, catheter, wire, bend, deflect, rotat+, angl+, wheel, position, chang+, variat+, quantity |

| **C.** **DOCUMENTS CONSIDERED TO BE RELEVANT** | | |
| --- | --- | --- |
| Category* | Citation of document, with indication, where appropriate, of the relevant passages | Relevant to claim No. |
| PX | CN 115715839 A (SHENZHEN EDGE MEDICAL CO., LTD.) 28 February 2023 (2023-02-28)<br>claims 1-10, description, paragraphs [0023]-[0122], and figures 1-12 | 1-6, 15-16 |
| PX | CN 115554568 A (SHENZHEN EDGE MEDICAL CO., LTD.) 03 January 2023 (2023-01-03)<br>claims 1-11, description, paragraphs [0024]-[0155], and figures 1-13 | 1-16 |
| A | CN 112336297 A (TONGJI UNIVERSITY) 09 February 2021 (2021-02-09)<br>description, paragraphs [0054]-[0192], and figures 1-12 | 1-16 |
| A | CN 101642364 A (OLYMPUS MEDICAL SYSTEMS CORP. (JP)) 10 February 2010 (2010-02-10)<br>entire document | 1-16 |
| A | CN 105188826 A (ST. JUDE MEDICAL LUXEMBOURG HOLDING SARL) 23 December 2015 (2015-12-23)<br>entire document | 1-16 |
| A | CN 109310287 A (INTUITIVE SURGICAL OPERATIONS, INC.) 05 February 2019 (2019-02-05)<br>entire document | 1-16 |

☑ Further documents are listed in the continuation of Box C.   ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **19 December 2023** | **22 December 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/130041** |

**C.    DOCUMENTS CONSIDERED TO BE RELEVANT**

| Category* | Citation of document, with indication, where appropriate, of the relevant passages | Relevant to claim No. |
|---|---|---|
| A | CN 114025699 A (CANON U.S.A, INC.) 08 February 2022 (2022-02-08)<br>    entire document | 1-16 |
| A | CN 114848144 A (SHANGHAI MICROPORT GUIDBOT CO., LTD.) 05 August 2022 (2022-08-05)<br>    entire document | 1-16 |
| A | US 2017209672 A1 (HANSEN MEDICAL, INC.) 27 July 2017 (2017-07-27)<br>    entire document | 1-16 |
| A | US 2020384243 A1 (BOSTON SCIENTIFIC SCIMED, INC.) 10 December 2020 (2020-12-10)<br>    entire document | 1-16 |

Form PCT/ISA/210 (second sheet) (July 2022)

## INTERNATIONAL SEARCH REPORT
### Information on patent family members

International application No.

**PCT/CN2023/130041**

| Patent document cited in search report | | | Publication date (day/month/year) | Patent family member(s) | | | Publication date (day/month/year) |
|---|---|---|---|---|---|---|---|
| CN | 115715839 | A | 28 February 2023 | None | | | |
| CN | 115554568 | A | 03 January 2023 | None | | | |
| CN | 112336297 | A | 09 February 2021 | None | | | |
| CN | 101642364 | A | 10 February 2010 | EP | 2151184 | A1 | 10 February 2010 |
| | | | | EP | 2151184 | B1 | 08 August 2012 |
| | | | | JP | 2010035768 | A | 18 February 2010 |
| | | | | US | 2010030023 | A1 | 04 February 2010 |
| | | | | US | 8597178 | B2 | 03 December 2013 |
| CN | 105188826 | A | 23 December 2015 | WO | 2014184665 | A2 | 20 November 2014 |
| | | | | EP | 2956199 | A2 | 23 December 2015 |
| | | | | EP | 2956199 | B1 | 25 October 2017 |
| | | | | EP | 3275498 | A1 | 31 January 2018 |
| | | | | EP | 3275498 | B1 | 22 May 2019 |
| | | | | JP | 2016518203 | A | 23 June 2016 |
| | | | | JP | 6343662 | B2 | 13 June 2018 |
| | | | | JP | 2018099529 | A | 28 June 2018 |
| | | | | JP | 6573687 | B2 | 11 September 2019 |
| | | | | US | 2016030715 | A1 | 04 February 2016 |
| | | | | US | 10010700 | B2 | 03 July 2018 |
| | | | | EP | 3275497 | A1 | 31 January 2018 |
| | | | | EP | 3275497 | B1 | 27 February 2019 |
| | | | | US | 2014324015 | A1 | 30 October 2014 |
| | | | | US | 9095682 | B2 | 04 August 2015 |
| CN | 109310287 | A | 05 February 2019 | US | 2022273377 | A1 | 01 September 2022 |
| | | | | EP | 3478149 | A1 | 08 May 2019 |
| | | | | US | 2019231449 | A1 | 01 August 2019 |
| | | | | US | 11344376 | B2 | 31 May 2022 |
| | | | | WO | 2018005928 | A1 | 04 January 2018 |
| CN | 114025699 | A | 08 February 2022 | WO | 2020243285 | A1 | 03 December 2020 |
| | | | | US | 2020375682 | A1 | 03 December 2020 |
| | | | | US | 11622828 | B2 | 11 April 2023 |
| | | | | JP | 2022535754 | A | 10 August 2022 |
| | | | | EP | 3976155 | A1 | 06 April 2022 |
| CN | 114848144 | A | 05 August 2022 | None | | | |
| US | 2017209672 | A1 | 27 July 2017 | US | 2023043963 | A1 | 09 February 2023 |
| | | | | US | 2020086087 | A1 | 19 March 2020 |
| | | | | US | 11452844 | B2 | 27 September 2022 |
| | | | | US | 2016015937 | A1 | 21 January 2016 |
| | | | | US | 9636483 | B2 | 02 May 2017 |
| | | | | EP | 2786721 | A2 | 08 October 2014 |
| | | | | EP | 2786721 | B1 | 13 May 2020 |
| | | | | US | 10493239 | B2 | 03 December 2019 |
| | | | | US | 2014276933 | A1 | 18 September 2014 |
| | | | | US | 9173713 | B2 | 03 November 2015 |
| US | 2020384243 | A1 | 10 December 2020 | EP | 3979940 | A1 | 13 April 2022 |
| | | | | WO | 2020247457 | A1 | 10 December 2020 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211497241

- CN 2022114972427